(19) Europäisches Patentamt
European Patent Office
Office européen des brevets

(11) **EP 3 722 777 A1**

(12) **EUROPÄISCHE PATENTANMELDUNG**

(43) Veröffentlichungstag:
**14.10.2020 Patentblatt 2020/42**

(51) Int Cl.:
**G01N 1/22** (2006.01)    **G01N 33/14** (2006.01)

(21) Anmeldenummer: **20163897.0**

(22) Anmeldetag: **18.03.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **12.04.2019   AT 503382019**

(71) Anmelder: **Anton Paar GmbH**
**8054 Graz-Straßgang (AT)**

(72) Erfinder:
• **GERMANN, Klaus**
  **8042 Graz-St. Peter (AT)**
• **GANGL, Elisabeth**
  **8053 Graz (AT)**
• **UMFER, Christof**
  **8041 Graz (AT)**

(74) Vertreter: **Wildhack & Jellinek**
**Patentanwälte**
**Landstraßer Hauptstraße 50**
**1030 Wien (AT)**

(54) **VERFAHREN ZUR MESSUNG DES SAUERSTOFFGEHALTS DES KOPFRAUMGASES IN EINEM GEBINDE**

(57)     Verfahren zur Messung des Sauerstoffgehalts und/oder des Sauerstoffpartialdrucks des Kopfraumgases in einem flüssigkeitsgefüllten Gebinde (6), insbesondere einer Getränkedose, einer Glas- oder einer Kunststoffflasche,
- wobei mit einem auf einem Anstechkopf (1) angeordneten, hohl ausgebildeten Piercer (2) eine Entnahmeöffnung in das Gebinde eingebracht wird, in die ein Entnahmerohr (3) eindringt und wobei die Entnahmeöffnung mittels am Piercer (2) oder dem Anstechkopf (1) angeordneten Dichtelementen luftdicht abgedeckt wird,
wobei das im Kopfraum (4) des Gebindes (6) befindliche Kopfraumgas mit einer Pumpe (9) über das Entnahmerohr (3) und/oder den hohl ausgebildeten Piercer (2) und/oder den Anstechkopf (1) in eine Sensoreinheit (8), umfassend eine Anzahl von Sensoren, und anschließend wieder zurück in den Kopfraum (4) des Gebindes (6) gepumpt wird und derart der Sauerstoffgehalt und/oder der Sauerstoffpartialdruck und/oder das Kopfraumvolumen des Kopfraumgases durch die Sensoreinheit (8) ermittelt wird.

Fig. 1

## Beschreibung

Die vorliegende Erfindung betrifft ein Verfahren gemäß dem Oberbegriff des Patentanspruchs 1 sowie eine Vorrichtung zur Durchführung des Verfahrens gemäß dem Oberbegriff des Patentanspruchs 11.

Für Getränkeabfüller ist die Kenntnis des Sauerstoffgehaltes in Gebinden, wie Getränkedosen, Flaschen und dergleichen, von großem Interesse, da dieser Einfluss auf die Haltbarkeit und den Geschmack des Getränks sowie bei Metallgebinden auf deren Korrosion hat. Um Rückschlüsse auf die Ursache eines eventuell vorhandenen Sauerstoffeintrags treffen zu können, ist es wichtig, den Sauerstoffgehalt in der Flüssigkeit und im Gasraum über der Flüssigkeit, dem sogenannten Kopfraum, getrennt zu bestimmen.

Man kann auf diese Weise feststellen, ob der Sauerstoff mit der Flüssigkeit ins Gebinde gelangt ist oder durch einen eventuell schlecht justierten Füllprozess. Die Messung im Gasraum ist besonders wichtig, da aufgrund der geringen Löslichkeit von Sauerstoff in wässrigen Flüssigkeiten ein signifikanter Anteil des Sauerstoffs des Gebindes im Kopfraum bzw. dem Kopfraumgas vorliegt. Die Messung der flüssigen Probe ist meist eine triviale Aufgabe, da die flüssige Probe über eine Schlauchleitung an einem Sauerstoffsensor vorbei aus dem Gebinde entnommen werden kann. Im Allgemeinen ist ausreichend Probe vorhanden, um solange am Sauerstoffsensor vorbeizuspülen, bis dieser nach einer Angleichzeit einen stabilen Wert anzeigt. Schwieriger ist die Situation bei der Messung des Kopfraumgases. Es sind meist nur wenige Milliliter Kopfraumgas vorhanden, wodurch es schwierig ist, dieses verwerfend am Sauerstoffsensor vorbeiströmen zu lassen.

Aus dem Stand der Technik sind eine Reihe von Messmethoden zur Messung der Sauerstoffkonzentration im Kopfraum bekannt. Mittels einer chemischen Methode wird das Kopfraumgas, meist bestehend aus $CO_2$, $N_2$ und $O_2$, durch eine Sodalösung geleitet, die das $CO_2$ absorbiert. Das verbleibende Volumen besteht dann aus $N_2$ und $O_2$ das mit einer Zusammensetzung der Umgebungsluft angenommen wird und so der Sauerstoffgehalt des Kopfraumgases berechnet wird. Nachteil der Methode ist, dass ein genauer Rückschluss auf den $O_2$ Gehalt nur möglich ist, wenn das Kopfraumgas tatsächlich der natürlichen Zusammensetzung von Luft mit ca. 20 % Sauerstoff entspricht. Im Allgemeinen ist dies aber nicht der Fall, da beim Abfüllen $N_2$ zum Spülen der Gebinde verwendet wird und dies das Verhältnis zwischen Sauerstoff und Stickstoff verändert. Ein weiterer Nachteil ist die mangelnde Automatisierbarkeit dieser Methode.

Weiters wird im Stand der Technik der Sauerstoffgehalt im Kopfraumgas über den in der Flüssigkeit gelösten Sauerstoff bestimmt. Voraussetzung dafür ist, dass das Gebinde bzw. der in dem Gebinde befindliche Sauerstoff in der Flüssigkeit und dem Kopfraumgas durch Schütteln ins Gleichgewicht gebracht wurde und dann der Sauerstoffgehalt der Flüssigkeit bestimmt wird. Nachteil der Methode ist, dass damit die Information verloren geht, ob der Sauerstoff über die Gasphase oder die Flüssigphase ins Gebinde gelangt ist. Ein weiterer Nachteil ist die aufwendige Schüttelprozedur, die mindestens 3 Minuten dauert.

Eine weitere bekannte Methode zur Bestimmung des Kopfraumgases ist die Messung direkt im Kopfraum durchzuführen. Bei dieser Methode wird das Gebinde angestochen und ein Sauerstoffsensor in den Kopfraum geführt. Nachteil der Methode ist, dass für die Bestimmung des Sauerstoffgehalts bei dieser Methode auch die Kenntnis der Temperatur am Messort erforderlich ist, da der Sensor stark temperaturabhängig ist. Konstruktiv ist es aus Platzmangel sehr schwierig, an dieser Stelle zusätzlich eine Temperaturmessung zu platzieren. Weiterer Nachteil ist, dass im Kopfraum keine Strömung herrscht, wodurch der Angleich des Sensors sehr langsam ist bzw. nicht vollständig stattfindet. Befindet sich, z.B bei Bier, Schaum im Kopfraum, funktioniert diese Messmethode nicht mehr zuverlässig.

Eine weitere bekannte Methode besteht darin Kopfraumgas zu extrahieren. Bei dieser Methode wird Kopfraumgas an einem Sauerstoffsensor vorbeiströmend entnommen. Wenn zuvor mit einer geeigneten Methode ein Gleichgewicht zwischen Flüssigkeit und Kopfraum hergestellt wurde, kann aus dieser Messung die Information über den Gesamtsauerstoffgehalt gewonnen werden. Nachteil der Methode ist, dass die Menge an Gas nicht ausreicht, um zuverlässig einen stabilen Angleich des Sauerstoffsensors zu erreichen. Ein weiterer Nachteil ist, dass nicht mehr feststellbar ist, ob sich der Sauerstoff ursprünglich in der Flüssigkeit oder im Kopfraum befunden hat.

Es ist daher Aufgabe der vorliegenden Erfindung ein Verfahren der eingangs genannten Art zu schaffen, dass die Nachteile des Standes der Technik behebt und somit eine genaue Messung des Sauerstoffgehalts im Kopfraumgas getrennt von dem Sauerstoffgehalt der Flüssigkeit ermöglicht und dabei die Dauer der Messung zu verringern.

Diese Aufgabe wird durch die kennzeichnenden Merkmale des Anspruchs 1 gelöst. Dabei ist vorgesehen, dass das im Kopfraum des Gebindes befindliche Kopfraumgas mit einer Pumpe über das Entnahmerohr und/oder den hohl ausgebildeten Piercer in eine Sensoreinheit, umfassend eine Anzahl von Sensoren, und anschließend wieder zurück in den Kopfraum des Gebindes gepumpt wird und derart der Sauerstoffgehalt und/oder der Sauerstoffpartialdruck, und insbesondere auch das Kopfraumvolumen, des Kopfraumgases durch die Sensoreinheit ermittelt wird.

Durch das erfindungsgemäße Verfahren wird erreicht, dass unabhängig von dem vorhandenen Kopfraumgas der Sauerstoffgehalt in diesem einfach bestimmt werden kann und unabhängig von dem im Kopfraum freien Volumen eine Vielzahl von Sensoren verwendet werden kann. Weiters müssen die Sensoren nicht in den Kopfraum direkt eingebracht werden, wodurch die Handhabung des Verfahrens vereinfacht wird. Ebenfalls steht immer genügend Kopfraumgas zur Verfügung, da dieses im Kreis bzw. wieder in den Kopfraum zurückgepumpt wird, wodurch eine genauere Bestimmung des Sauerstoffgehalts und/oder des Sauerstoffpartialdrucks und des Kopfraumvolumens erreicht wird.

Besonders vorteilhafte Ausführungsformen des erfindungsgemäßen Verfahrens werden durch die Merkmale der abhängigen Ansprüche näher definiert:

Vorteilhaft kann vorgesehen sein, dass, insbesondere bei der Vermessung von Gebinden mit schäumenden Flüssigkeiten, im Kopfraum des Gebindes ein Schaum erzeugt wird, in dem das Kopfraumgas oder ein Teil des Kopfraumgases gebunden ist, wobei der erzeugte Schaum in die Sensoreinheit und anschließend wieder in den Kopfraum des Gebindes zurückgeführt wird. So kann beispielsweise, auch wenn nur ein geringer Anteil des Kopfraumgases vorhanden ist, das in dem Schaum gebundene Kopfraumgas bzw. der darin enthaltene Sauerstoff einfach ermittelt werden, da aufgrund der schlechten Löslichkeit von Sauerstoff in wässrigen Substanzen der Sauerstoffgehalt im Schaum eine sehr gute Entsprechung des Sauerstoffgehalts im Kopfraum ist.

Zur einfachen Bestimmung des Sauerstoffgehalts und/oder des Sauerstoffpartialdrucks und/oder des Kopfraumvolumens kann vorgesehen sein, dass die Sensoreinheit einen Sauerstoffsensor zur Messung des Sauerstoffgehalts und/oder des Sauerstoffpartialdrucks des Kopfraumgases aufweist, wobei insbesondere der Sauerstoffgehalt und/oder das Kopfraumvolumen durch zusätzliche Messung des Drucks mittels eines Drucksensors und/oder Messung der Temperatur mittels eines Temperatursensors, vorzugsweise bei einer bewirkten Volumenänderung des Kopfraumgases, ermittelt wird.

Da eine Vielzahl von Sensoren temperaturempfindlich sind bzw. einen Temperaturangleich an das zu vermessende Medium erfordern, um eine höchste Genauigkeit zu erreichen, kann vorgesehen sein, dass das Kopfraumgas so oft aus dem Kopfraum in die Sensoreinheit, insbesondere an dem Temperatursensor vorbei, und anschließend wieder in den Kopfraum zurück gepumpt wird, bis die Sensoreinheit, insbesondere der Drucksensor und der Temperatursensor und der Sauerstoffsensor, und/oder das Kopfraumgas eine stabile, vorzugsweise gleiche, Temperatur erreichen. Durch das mehrmalige Umpumpen des Kopfraumgases durch die Sensoreinheit wird erreicht, dass die einzelnen Sensoren der Sensoreinheit die Temperatur des Kopfraumgases annehmen können bzw. an diese angeglichen werden und die Messungen des Kopfraumgases dann in einem angeglichenen Temperaturbereich erfolgen kann. Weiters wird derart der Temperaturangleich der Sensoren bzw. der Sensoreinheit an die Temperatur des Kopfraumgases beschleunigt. Darüber hinaus haben sonstige Angleichsprozesse wie z.B. Diffusionsprozesse im Sauerstoffsensor hinreichend Zeit für einen vollständigen Angleich.

Um den Sauerstoffgehalt der Probenflüssigkeit ebenfalls ermitteln zu können, kann vorgesehen sein, dass nach der Messung des Sauerstoffgehalts des Kopfraumgases das Entnahmerohr in die in dem Gebinde befindliche Flüssigkeit abgesenkt wird und anschließend die Flüssigkeit aus dem Gebinde entnommen und in die Sensoreinheit geleitet wird und derart der in der Flüssigkeit befindliche Sauerstoffgehalt ermittelt wird.

Vorteilhaft kann vorgesehen sein, dass die Sensoreinheit eine Anzahl weiterer Sensoren, insbesondere einen $CO_2$-Sensor, einen Alkoholsensor und/oder einen Zuckersensor, aufweist, wobei mittels der weiteren Sensoren der $CO_2$ Gehalt und/oder der Alkoholholgehalt und/oder der Zuckergehalt der in dem Gebinde befindlichen Flüssigkeit bestimmt wird. Durch die Anordnung unterschiedlicher Sensoren innerhalb der Sensoreinheit bzw. innerhalb der Messanordnung können unterschiedlichste Parameter der Probenflüssigkeit bzw. des Kopfraumgases ermittelt werden, sodass eine vollständige Analyse der Probenflüssigkeit und/oder des Kopfraumgases erreicht werden kann. Alternativ kann vorgesehen sein, dass zusätzliche Sensoren außerhalb der Sensoreinheit in der Leitung bzw. der Ringleitung angeordnet sind.

Der Füllstand der Probenflüssigkeit innerhalb des Gebindes kann je nach Gebinde und Füllvolumen abweichen. So kann das Kopfraumgas, wie beispielsweise bei Getränkedosen immer wieder auftretend, je nach Anordnung des Gebindes nicht direkt zugänglich sein und daher nicht direkt über die Entnahmeöffnung abgepumpt werden. Um die Verfügbarkeit des Kopfraumgases leichter zu bewirken bzw. den Kopfraum zu erweitern, kann vorgesehen sein, dass vor der Messung des Kopfraumgases der Flüssigkeitsspiegel in dem Gebinde über das Entnahmerohr derart abgesenkt wird, dass der Kopfraum eine direkte Verbindung zur Entnahmeöffnung erhält.

Um etwaige vorhandenen Sauerstoff bzw. Verunreinigungen aus der Probenanordnung auszubringen bzw. eine Verfälschung der Messergebnisse besser verhindern zu können, kann vorgesehen sein, dass vor der Messung der Piercer, der Anstechkopf, die Sensoreinheit, die Pumpe, die Ringleitung und/oder das Entnahmerohr mit einem Spülmedium, insbesondere Stickstoff, gespült und derart von Sauerstoff und/oder Probenrückständen befreit werden.

Vorteilhaft kann vorgesehen sein, dass vor dem Anstechen des Gebindes der Druck in dem Piercer und/oder dem Anstechkopf und/oder in der Ringleitung, insbesondere durch Einbringung von Stickstoffgas, an den Innendruck des Gebindes angeglichen wird, sodass ein Aufschäumen der Probenflüssigkeit verhindert wird. So kann eine Schaumbildung einfach vermieden und ein unerwünschtes Austreten des Kopfraumgases unterbunden werden.

Optional kann vorgesehen seine, dass nach Anstechen des Gebindes der Sauerstoffsensor und/oder Temperatursensor, insbesondere über das Entnahmerohr oder den Piercer, in den Kopfraum des Gebindes eingebracht wird und der Sauerstoffgehalt und/oder die Temperatur des Kopfraumgases in dem Kopfraum ermittelt wird. So kann durch das Umpumpen ein rascher Angleich der Sensoren erreicht werden und eine Schichtbildung des Kopfraumgases vorteilhaft verhindert werden.

Es ist weiters Aufgabe der Erfindung, eine Vorrichtung zu schaffen, mit der der Sauerstoffgehalt des Kopfraumgases einfach ermittelt werden kann.

Diese Aufgabe wird durch die Merkmale des Anspruchs 11 gelöst. Dabei ist erfindungsgemäß vorgesehen, dass die Vorrichtung eine Pumpe und eine Ringleitung aufweist, wobei innerhalb der Ringleitung eine Sensoreinheit angeordnet ist, mit der Sauerstoffgehalt und/oder der Sauerstoffpartialdruck, und insbesondere auch das Kopfraumvolumen, des Kopfraumgases des Gebindes ermittelbar ist, wobei die Ringleitung derart ausgebildet ist, dass das Kopfraumgas des Gebindes über den hohl ausgebildeten Piercer oder den Anstechkopf mittels der Pumpe entnehmbar und über die Ringleitung, insbesondere über das Entnahmerohr, in den Kopfraum des Gebindes zurückleitbar ist.

Mit der erfindungsgemäßen Vorrichtung kann der Sauerstoffgehalt bzw. der Sauerstoffpartialdruck und/oder das Kopfraumvolumen einfach bestimmt werden und es wird eine hohe Messgenauigkeit erreicht.

Vorteilhaft kann vorgesehen sein, dass die Sensoreinheit einen Sauerstoffsensor und/oder einen Drucksensor und/oder einen Temperatursensor und/oder einen CO2-Sensor und/oder einen Alkoholsensor und/oder einen Zuckersensor aufweist, wobei der Sauerstoffsensor insbesondere als optochemischer Sensor nach dem Fluorescence Quenching Prinzip oder als elektrochemischer Sauerstoffsensor ausgebildet ist.

Eine effektive Umwälzung des Kopfraumgases kann einfach erreicht werden, indem die Pumpe als Umwälzpumpe, bevorzugt als Membranpumpe, Schlauchquetschpumpe, Kolbenpumpe, Zahnradpumpe, Schneckenpumpe, Schaufelradpumpe oder Spritzenpumpe, ausgebildet ist.

Um ein Spülgas, wie beispielsweise Stickstoff, einfach in die Vorrichtung einbringen zu können, kann vorgesehen sein, dass die Vorrichtung eine Anzahl von in der Ringleitung integrierten Ventilen aufweist, wobei die Ventile derart in der Ringleitung angeordnet sind, dass eine automatische Reinigung der Vorrichtung, insbesondere der Ringleitung, über die Ventile erfolgen kann.

Da der zur Verfügung stehende Anstechbereich vor allem bei Gebinden mit engem Hals gering ist, ist eine platzsparende Anordnung bzw. Ausbildung des Piercers und des Entnahmerohrs vorteilhaft. So kann erfindungsgemäß vorgesehen sein, dass der Piercer hohl ausgebildet ist, wobei das Entnahmerohr durch den Piercer hindurch in die Entnahmeöffnung einbringbar ist.

Weitere Vorteile und Ausgestaltungen der Erfindung ergeben sich aus der Beschreibung und den beiliegenden Zeichnungen.

Die Erfindung ist im Folgenden anhand von besonders vorteilhaften, aber nicht einschränkend zu verstehenden Ausführungsbeispielen in den Zeichnungen schematisch dargestellt und wird unter Bezugnahme auf die Zeichnungen beispielhaft beschrieben:

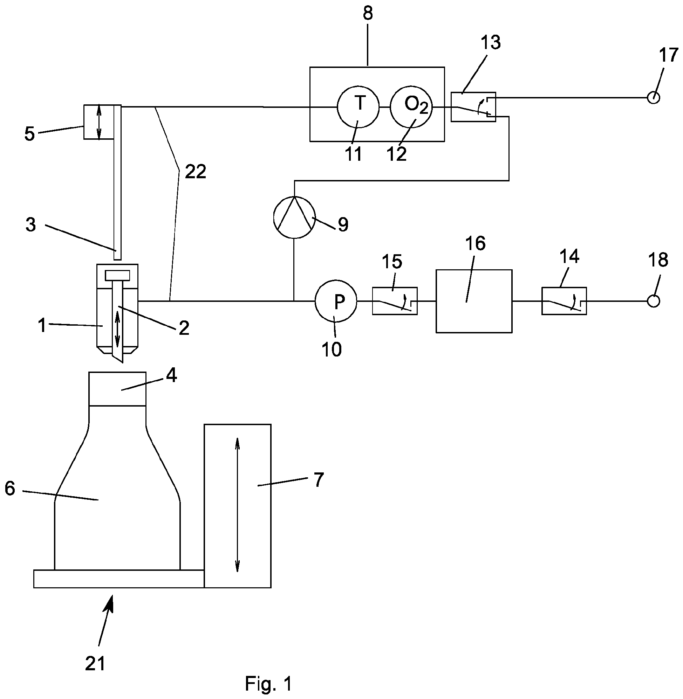

Fig. 1 zeigt eine erfindungsgemäße Vorrichtung in schematischer Darstellung.

In Fig. 1 ist eine erfindungsgemäße Vorrichtung zur Bestimmung des Sauerstoffgehalts des Kopfraumgases eines Gebindes in schematischer Ansicht dargestellt. Die Vorrichtung umfasst einen Anstechkopf 1, auf dem ein hohl ausgebildeter Piercer 2 angeordnet ist. Der Piercer 2 ist bei dieser Ausführungsform ähnlich einer Nadel ausgebildet und kann innerhalb des Anstechkopfs 1 entlang der dargestellten Pfeile verstellt werden. Die Vorrichtung umfasst weiters eine Probenaufnahme 21, in die ein Gebinde 6, bei der in Fig. 1 dargestellten Ausführungsform eine Flasche, eingebracht ist. Mittels eines Antriebs 7 kann das Gebinde 6 in Richtung des Anstechkopfs 1 verstellt werden, wodurch der Anstechkopf 1 auf das verschlossene Gebinde 6 aufgelegt bzw. angebracht werden kann. Die Vorrichtung umfasst weiters ein Entnahmerohr 3, das bei dieser Ausführungsform konzentrisch zu dem Piercer 2 angeordnet ist. Der Piercer 2 ist bei dieser Ausführungsform hohl ausgebildet, sodass das Entnahmerohr 3 in den Piercer 2 eindringt und durch diesen hindurch in den Kopfraum 4 gebracht werden kann. Das Entnahmerohr 3 wird dabei mittels eines Antriebes 5 verstellt. Optional kann auch vorgesehen sein, dass die Probenaufnahme 21, der Piercer 2, der Anstechkopf 1 und/oder das Entnahmerohr 3 händisch bzw. gesteuert durch einen Antrieb mit Steuerung verstellt werden können. An dem dem Anstechkopf 1 entfernten Ende des Entnahmerohrs 3 beginnt eine Ringleitung 22, die wieder in den Anstechkopf 1 zurückführt bzw. wieder in diesen mündet. In der Ringleitung 22 ist eine Sensoreinheit 8 und eine Pumpe 9 integriert. Mittels der Pumpe 9 kann eine in dem Gebinde 6 befindliche Probenflüssigkeit oder das im Kopfraum 4 des Gebindes 6 befindliche Kopfraumgas über das Entnahmerohr 3 entnommen und so der Sensoreinheit 8 zugeführt werden. Über die Sensoreinheit 8 wird die Probenflüssigkeit oder das Kopfraumgas wieder durch die Pumpe 9 in den Anstechkopf und so wieder in das Gebinde 6 bzw. in den Kopfraum 4 des Gebindes 6 zurückgeführt. Optional kann auch vorgesehen sein, dass das Kopfraumgas und/oder die Probenflüssigkeit über den Anstechkopf entnommen werden und durch das Entnahmerohr 3 wieder in den Kopfraum 4 des Gebindes 6 zurückgeführt werden.

Die Sensoreinheit 8 umfasst bei dieser Ausführungsform einen Temperatursensor 11 sowie einen Sauerstoffsensor 12. Die Vorrichtung umfasst weiters einen Drucksensor 10, mit dem der in der Ringleitung 22 bzw. dem Kopfraum 4 des Gebindes 6 anstehende Druck ermittelt werden kann.

Im Folgenden wird das erfindungsgemäße Verfahren anhand der Ausführungsform der Fig. 1 beispielhaft beschrieben:

Bei dem erfindungsgemäßen Verfahren wird in einem ersten Schritt das Gebinde 6 oder der Anstechkopf 1 verstellt, sodass der Anstechkopf 1 an dem Gebinde 6, beispielsweise an dem Deckel einer Flasche, ansteht. Anschließend wird der Piercer 2 in Richtung des Gebindes verstellt und durchstößt beispielsweise den Deckel der Flasche und erzeugt so eine Entnahmeöffnung in dem Gebinde 6. Mittels an dem Piercer 2 und/oder an dem Anstechkopf 1 angeordneten Dichtelementen wird die Entnahmeöffnung luftdicht abgedeckt, sodass aus der Umgebung kein Fremdgas in die Vorrichtung bzw. die Ringleitungen 22 oder den Kopfraum 4 des Gebindes 6 eindringen kann bzw. kein Gas aus dem Gebinde 6 entweichen kann. Die Dichtelemente des Anstechkopfs 1 bzw. des Piercers 2 dichten dabei das Gebinde 6 sowie die Ringleitungen gegen die Umgebung der Vorrichtung luftdicht ab. Das Entnahmerohr 3 wird anschließend in den Kopfraum 4 des Gebindes 6 abgesenkt, sodass das Entnahmerohr 3 in den Kopfraum 4 eindringt ohne in die Flüssigkeit bzw. Probenflüssigkeit des Gebindes 6 einzutauchen. Über das Entnahmerohr 3 wird dann mittels der Pumpe 9 das Kopfraumgas aus dem Kopfraum 4 abgepumpt und über die Ringleitung 22 der Sensoreinheit 8 zugeführt. In der Sensoreinheit 8 wird mittels des Temperatursensors 11 und des Sauerstoffsensors 12 der Sauerstoffgehalt sowie die Temperatur des Kopfraumgases bestimmt. Mittels des Drucksensors 10 wird weiters der im Kopfraum 4 anstehende Druck ermittelt und dann beispielsweise über die Gasgleichungen das Volumen des Kopfraumgases bestimmt. Über die Ringleitung 22 wird dann durch die Pumpe 9 das Kopfraumgas von der Sensoreinheit 8 wieder über den Anstechkopf 1 in den Kopfraum 4 des Gebindes 6 zurückbefördert. Durch das Umpumpen des Kopfraumgases über die Ringleitungen wird so ein Kreislauf des Kopfraumgases innerhalb der Vorrichtung bewirkt, sodass das Kopfraumgas einfach oder auch mehrfach an der Sensoreinheit 8 bzw. den Sensoren der Vorrichtung vorbei gepumpt werden kann. Durch das Vorbeipumpen des Kopfraumgases an der Sensoreinheit 8 bzw. den Sensoren wird der Temperaturangleich der Sensoren verbessert, sodass die Bestimmung des Sauerstoffgehalts, des Sauerstoffpartialdrucks und/oder des Kopfraumvolumens beschleunigt und genauer bestimmt werden kann. Weiters wird der Abschluss sensorspezifischer Angleichvorgänge, wie z.B. Diffusionsvorgänge im Sauerstoffsensor, vollständig abgewartet.

Nach der Bestimmung des Sauerstoffgehalts des Kopfraumgases bzw. des Kopfraumvolumens oder des Sauerstoffpartialdrucks kann das Entnahmerohr 3 aus dem Kopfraum 4 weiter in die Probenflüssigkeit des Gebindes abgesenkt werden. Anschließend wird die Probenflüssigkeit zur Sensoreinheit 8 gepumpt und auch der Sauerstoffgehalt, die Temperatur bzw. der Druck der Probenflüssigkeit bestimmt.

Da Gebinde 6 mit schäumenden Flüssigkeiten dazu neigen nach dem Anstechen mittels des Piercers 2, insbesondere bei schnellem Einlegen in den Probenhalter 21, zu schäumen, kann beispielsweise die Schaumbildung im Kopfraum 4 des Gebindes 6 absichtlich verstärkt werden bzw. auch erst ein Schaum erzeugt werden, indem das Kopfraumgas oder ein Teil des Kopfraumgases gebunden ist. Anschließend kann der erzeugte Schaum mittels der Pumpe 9 über die Ringleitungen 22 der Sensoreinheit 8 zugeführt werden und so der Sauerstoffgehalt des Schaums ermittelt werden. Da der Sauerstoffgehalt des Schaums dem des Kopfraumes 4 bzw. des Kopfraumgases entspricht, können so der Sauerstoffgehalt des Kopfraumgases bestimmt werden.

Da die Sensoren meist ein temperaturabhängiges Messverhalten sowie ein durch unterschiedliche physikalische Effekte verursachtes Angleichverhalten haben, ist es vorteilhaft, dass die Sensoren, insbesondere der Sauerstoffsensor 12 und der Temperatursensor 11, an die Temperatur des Kopfraumgases bzw. der Probenflüssigkeit angeglichen werden bzw. sonstige Angleichvorgänge abgewartet werden. Um diesen Angleich rasch durchführen zu können, wird optional das Kopfraumgas 4 mehrfach an der Sensoreinheit 8 bzw. an den Sensoren vorbeigepumpt und so der Angleich beschleunigt. Durch die Umwälzung des Kopfraumgases bzw. durch das mehrfache Umpumpen des Kopfraumgases können so auch geringe Mengen des Kopfraumgases mittels der Sensoreinheit 8 vermessen werden bzw. auch bei geringen Mengen des Kopfraumgases ein rascher Angleich der Sensoren an das Kopfraumgas und die Probenflüssigkeit erreicht werden.

Die Vorrichtung umfasst weiters ein in der Ringleitung 22 angeordnetes Ventil 13, das mit einer Leitung, die in die Umgebung an einer Spülöffnung 17 führt, verbunden ist. Über die Spülöffnung 17 kann beispielsweise ein Spülgas wie Stickstoff oder eine Reinigungslösung in die Ringleitung bzw. zu der Sensoreinheit 8 bzw. der Pumpe 9 und den Sensoren gelangen und so Probenrückstände oder Restsauerstoff aus der Vorrichtung ausgespült werden.

Optional kann vorgesehen sein, dass, wie in Fig. 1 dargestellt, die Vorrichtung eine Anzahl von weiteren Ventilen 14, 15 sowie einen Speichervolumen 16 umfasst. Das Speichervolumen 16 ist über das Ventil 15 mit dem Drucksensor 10 verbunden und über ein weiteres Ventil 14 mit der Umgebung der Vorrichtung verbunden.

Da das ursprünglich nur im Kopfraum 3 befindliche Kopfraumgas beim Öffnen des Gebindes 6 durch den Piercer 2 sich in der Ringleitung 22 verteilen kann, werden niedrigere $O_2$ Konzentrationen gemessen als im Kopfraum 4 des ursprünglich geschlossenen Gebindes 6 vorhanden war. Dieser systematische Fehler wird rechnerisch korrigiert. Dazu ist die Kenntnis des Pumpkreisvolumens bzw. das Volumen der Ringleitungen 22 und der daran angeschlossenen Komponenten notwendig und die Kenntnis des Kopfraumvolumens. Das Kopfraumvolumen wird im Zuge des Messablaufs durch die Sensoreinheit 8 bzw. den Drucksensor 10 und den Temperatursensor 11 messtechnisch bestimmt und/oder über die Anwendung der Gasgesetze ermittelt.

Dazu ist in der Vorrichtung der Ausführungsform der Fig. 1 ein leeres Speichervolumen 16 integriert. In einem ersten Schritt vor dem Anstechen des Gebindes 6 wird das Ventil 14, das über einen Lufteingang 18 mit der Umgebung der Vorrichtung verbunden ist, geöffnet und das Speichervolumen 16 auf Umgebungs-Luftdruck gebracht. Wenn auch das Ventil 15, das das Speichervolumen mit der Ringleitung 22 verbindet, geöffnet ist, kann ein erster Luftdruck p1 gemessen werden. Nun werden die Ventile 14 und 15 geschlossen. Nach dem Anstechen des Gebindes 6 durch den Piercer 2 wird der Druck p2 gemessen, der sich aus der Kombination des Drucks des angestochenen Gebindes 6 und dem im Anstechkopf 1 und den Ringleitungen 22 anliegenden Druck ergibt. Dann wird das Ventil 15 geöffnet und der sich dann einstellende Mischdruck p3 gemessen. Bei bekanntem Speichervolumen 16 kann nun das Kopfraumvolumen $V_{Headspace}$ unter Anwendung des Boyle Mariotte'schen Gesetzes (Gleichung 1) berechnet werden.

$$V_{Headspace} = V_{Expansion} \times \frac{p_3 - p_2}{p_1 - p_3} - V_{Apparat} \quad \text{Gleichung 1}$$

Da die Expansion weder rein isotherm noch rein adiabatisch abläuft, stellt das Ergebnis nur eine gute Näherung dar.

Expansionsvolumen $V_{Expansion}$ und Apparatvolumen $V_{Apparat}$ also das in der Vorrichtung bzw. in der Ringleitung 22, dem Entnahmerohr 3, dem Piercer 2 und dem Anstechkopf 1 befindliche Volumen, können aus der Konstruktion bestimmt werden, besser ist allerdings, wenn mit verschiedenen bekannten Kopfraumvolumina $V_{Headspace}$ eine Messreihe durchgeführt und daraus die $V_{Expansion}$ und $V_{Apparat}$ berechnet werden. Die beiden Werte enthalten dann neben Geometrieinformationen Korrekturen für Abweichungen vom isothermen Verhalten und können so ein noch genaueres Ergebnis der Messung ermöglichen.

Die gemessene Sauerstoffkonzentration kann dann mit den bekannten Volumina anhand der Gleichung 2 korrigiert werden.

$$O_{2,corr} = O_{2,gemessen} \times \frac{V_{Apparat} + V_{Headspace}}{V_{Headspace}} \quad \text{Gleichung 2}$$

Alternativ kann man am Beginn der Messung das Speichervolumen 16 auch auf einen höheren Druck als den, der im Gebinde 6 herrscht, bringen. Dazu wird der Anstechkopf 1 vor dem Anstechen mit dem Piercer 2 zum Gebinde 6 abgedichtet. Dann werden die Ventile 14 und 15 geöffnet, sodass im gesamten Bereich zwischen dem Lufteingang 18 und Anstechkopf 1 derselbe Druck herrscht. Dieser Druck wird mit dem Drucksensor 10 gemessen. Anschließend werden die Ventile 14 und 15 geschlossen und der Druck damit im Speichervolumen 16 "eingesperrt". Das restliche Verfahren zur Messung der Sauerstoffkonzentration wird dann analog zu dem oben beschriebenen Verfahren durchgeführt.

Alternativ kann vorgesehen sein, dass das Kopfraumgas bzw. die Probenflüssigkeit über den Piercer 2 in die Ringleitung 22 abgepumpt wird oder das Entnahmerohr 3 direkt an den Piercer 2 anschließt. Alternativ kann der Piercer 2 nach dem Öffnen bzw. Durchstechen des Gebindes 6 in dem Kopfraum 4 verbleiben und über diesen das Kopfraumgas bzw. die Probenflüssigkeit in die Ringleitung 22 gepumpt werden.

Optional kann die Sensoreinheit 8 oder die Vorrichtung auch eine Anzahl weiterer Sensoren beispielsweise einen $CO_2$-Sensor, einen Alkoholsensor, einen Zuckersensor und/oder weitere Sensoren aufweisen, die in der Ringleitung 22 oder der Sensoreinheit 8 integriert sind. Mittels der weiteren Sensoren kann beispielsweise der $CO_2$-Gehalt oder der Alkoholgehalt oder der Zuckergehalt der Probenflüssigkeit bestimmt werden und so weitere Parameter der Probenflüssigkeit ermittelt werden. Die weiteren Sensoren können optional auch über die Öffnung 17 mit der Probenflüssigkeit befüllt werden. Die weiteren Sensoren können beispielsweise bei der Produktion von Getränken wie Bier oder Limonaden weitere Informationen liefern, sodass mittels der erfindungsgemäßen Vorrichtung die Qualitätskontrolle des Abfüllprozesses oder des Produktionsprozesses einfach überwacht werden kann.

Der Sauerstoffsensor 12 kann insbesondere als optochemischer Sensor nach dem Fluorescence Quenching Prinzip oder beispielsweise als elektrochemischer Sauerstoffsensor ausgebildet sein. Optional zu der in Fig. 1 dargestellten Ausführungsform kann die Sensoreinheit auch nur einen Sauerstoffsensor 12 umfassen, mit dem der Sauerstoffgehalt des Kopfraumgases und/oder der Probenflüssigkeit bestimmt wird.

Die Pumpe 9, der in Fig. 1 dargestellten Ausführungsform kann beispielsweise als Umwälzpumpe, insbesondere als Membranpumpe, Schlauchquetschpumpe, Kolbenpumpe, Zahnradpumpe, Schneckenpumpe, Schaufelradpumpe oder Spritzenpumpe ausgebildet sein

Optional können der Antrieb 7 bzw. die Verstellmechanismen des Piercers 2 und des Entnahmerohrs 3 händisch oder auch anders angetrieben werden und so die Verstellung der einzelnen Teile zueinander bewirkt werden.

Alternativ kann vorgesehen sein, dass anstelle der Anordnung des Sauerstoffsensors 12 innerhalb der Ringleitung 22 bzw. der Sensoranordnung 8, der Sauerstoffsensor 12 über das Entnahmerohr 3 oder den Piercer 2 in den Kopfraum 4 des Gebindes 6 eingebracht wird. Das Kopfraumgas kann dann über die Ringleitung 22 umgepumpt werden und so der Angleich der Sensoren, durch die Umwälzung des Kopfraumgases verbessert bzw. beschleunigt werden.

Optional kann ebenfalls der Temperatursensor 11 in den Kopfraum 4 eingebracht werden.

**Patentansprüche**

1. Verfahren zur Messung des Sauerstoffgehalts und/oder des Sauerstoffpartialdrucks des Kopfraumgases in einem flüssigkeitsgefüllten Gebinde (6), insbesondere einer Getränkedose, einer Glas- oder einer Kunststoffflasche,

   - wobei mit einem auf einem Anstechkopf (1) angeordneten, hohl ausgebildeten Piercer (2) eine Entnahmeöffnung in das Gebinde eingebracht wird, in die ein Entnahmerohr (3) eindringt und wobei die Entnahmeöffnung mittels am Piercer (2) oder dem Anstechkopf (1) angeordneten Dichtelementen luftdicht abgedeckt wird, **dadurch gekennzeichnet, dass** das im Kopfraum (4) des Gebindes (6) befindliche Kopfraumgas mit einer Pumpe (9) über das Entnahmerohr (3) und/oder den hohl ausgebildeten Piercer (2) und/oder den Anstechkopf (1) in eine Sensoreinheit (8), umfassend eine Anzahl von Sensoren, und anschließend wieder zurück in den Kopfraum (4) des Gebindes (6) gepumpt wird und derart der Sauerstoffgehalt und/oder der Sauerstoffpartialdruck und/oder das Kopfraumvolumen des Kopfraumgases durch die Sensoreinheit (8) ermittelt wird.

2. Verfahren nach Anspruch 1, **dadurch gekennzeichnet, dass**, insbesondere bei der Vermessung von Gebinden (6) mit schäumenden Flüssigkeiten, im Kopfraum (4) des Gebindes (6) ein Schaum erzeugt wird, in dem das Kopfraumgas oder ein Teil des Kopfraumgases gebunden ist, wobei der erzeugte Schaum in die Sensoreinheit (8) und anschließend wieder in den Kopfraum (4) des Gebindes (6) zurückgeführt wird.

3. Verfahren nach einem der Ansprüche 1 oder 2, **dadurch gekennzeichnet, dass** die Sensoreinheit (8) einen Sauerstoffsensor (12) zur Messung des Sauerstoffgehalts und/oder des Sauerstoffpartialdrucks des Kopfraumgases aufweist, wobei insbesondere der Sauerstoffgehalt und/oder das Kopfraumvolumen durch zusätzliche Messung des Drucks mittels eines Drucksensors (10) und/oder Messung der Temperatur mittels eines Temperatursensors (11), vorzugsweise bei einer bewirkten Volumenänderung des Kopfraumgases, ermittelt wird.

4. Verfahren nach einem der vorangehenden Ansprüche, **dadurch gekennzeichnet, dass** das Kopfraumgas so oft aus dem Kopfraum (4) in die Sensoreinheit (8), insbesondere an dem Temperatursensor (11) und/oder Sauerstoffsensor (12) vorbei, und anschließend wieder in den Kopfraum zurückgepumpt wird bis die Sensoreinheit (8), insbesondere der Drucksensor (10) und/oder der Temperatursensor (11) und/oder der Sauerstoffsensor (12), und/oder das Kopfraumgas eine stabile, vorzugsweise gleiche, Temperatur erreichen.

5. Verfahren nach einem der vorangehenden Ansprüche, **dadurch gekennzeichnet, dass** nach der Messung des Sauerstoffgehalts des Kopfraumgases das Entnahmerohr (3) in die in dem Gebinde (6) befindliche Flüssigkeit abgesenkt wird und anschließend die Flüssigkeit aus dem Gebinde (6) entnommen und in die Sensoreinheit (8) geleitet wird und derart der in der Flüssigkeit befindliche Sauerstoffgehalt ermittelt wird.

6. Verfahren nach einem der vorangehenden Ansprüche, **dadurch gekennzeichnet, dass** die Sensoreinheit (8) eine Anzahl weiterer Sensoren, insbesondere einen $CO_2$-Sensor, einen Alkoholsensor und/oder einen Zuckersensor, aufweist, wobei mittels der weiteren Sensoren der $CO_2$ Gehalt und/oder der Alkoholholgehalt und/oder der Zuckergehalt der in dem Gebinde befindlichen Flüssigkeit bestimmt wird.

7. Verfahren nach einem der vorangehenden Ansprüche, **dadurch gekennzeichnet, dass** vor der Messung des Kopfraumgases der Flüssigkeitsspiegel in dem Gebinde (6) über das Entnahmerohr (3) derart abgesenkt wird, dass der Kopfraum eine direkte Verbindung zur Entnahmeöffnung erhält.

8. Verfahren nach einem der vorangehenden Ansprüche, **dadurch gekennzeichnet, dass** vor der Messung der Piercer (2), der Anstechkopf (1), die Sensoreinheit (8), die Pumpe (9), die Ringleitung (22) und/oder das Entnahmerohr (3) mit einem Spülmedium, insbesondere Stickstoff, gespült und derart von Sauerstoff und/oder Probenrückständen befreit werden.

9. Verfahren nach einem der vorangehenden Ansprüche, **dadurch gekennzeichnet, dass** vor dem Anstechen des Gebindes (6) der Druck in dem Piercer (3) und/oder dem Anstechkopf (1) und/oder in der Ringleitung (22), insbesondere durch Einbringung von Stickstoffgas, an den Innendruck des Gebindes (6) angeglichen wird, sodass ein Aufschäumen der Probenflüssigkeit verhindert wird.

10. Verfahren nach einem der vorangehenden Ansprüche, **dadurch gekennzeichnet, dass** nach Anstechen des Gebindes (6) der Sauerstoffsensor (12) und/oder Temperatursensor (11), insbesondere über das Entnahmerohr 3 oder den Piercer (2), in den Kopfraum (4) des Gebindes eingebracht und der Sauerstoffgehalt und/oder die Temperatur des Kopfraumgases in dem Kopfraum (4) ermittelt wird.

11. Vorrichtung zur Bestimmung des Sauerstoffgehalts des Kopfraumgases in einem flüssigkeitsgefüllten Gebinde (6), insbesondere einer Getränkedose, einer Glas- oder einer Kunststoffflasche, insbesondere zur Durchführung des Verfahrens nach einem der Ansprüche 1 bis 10, wobei die Vorrichtung einen Anstechkopf (1) aufweist, an dem ein, insbesondere hohl ausgebildeter, Piercer (2) und ein Entnahmerohr (3) angeordnet sind, **dadurch gekennzeichnet, dass** die Vorrichtung eine Pumpe (6) und eine Ringleitung (22) aufweist, wobei innerhalb der Ringleitung (22) eine Sensoreinheit (8) angeordnet ist, mit der der Sauerstoffgehalt und/oder der Sauerstoffpartialdruck, und insbesondere auch das Kopfraumvolumen, des Kopfraumgases des Gebindes ermittelbar ist, wobei die Ringleitung (22) derart ausgebildet ist, dass das Kopfraumgas des Gebindes (6) über den hohl ausgebildeten Piercer (2) oder den Anstechkopf (1) mittels der Pumpe (6) entnehmbar und über die Ringleitung (22), insbesondere über das Entnahmerohr (3), in den Kopfraum (4) des Gebindes (6) zurückleitbar ist.

12. Vorrichtung nach Anspruch 11, dadurch gegenzeichnet, dass die Sensoreinheit (8) einen Sauerstoffsensor (12) und/oder einen Drucksensor (10) und/oder einen Temperatursensor (11) und/oder einen $CO_2$-Sensor und/oder einen Alkoholsensor und/oder einen Zuckersensor aufweist, wobei der Sauerstoffsensor (12) insbesondere als optochemischer Sensor nach dem Fluorescence Quenching Prinzip oder als elektrochemischer Sauerstoffsensor ausgebildet ist.

13. Vorrichtung nach einem der Ansprüche 11 oder 12, **dadurch gekennzeichnet, dass** die Pumpe (9) als Umwälzpumpe, bevorzugt als Membranpumpe, Schlauchquetschpumpe, Kolbenpumpe, Zahnradpumpe, Schneckenpumpe, Schaufelradpumpe oder Spritzenpumpe, ausgebildet ist.

14. Vorrichtung nach einem der Ansprüche 1 bis 13, **dadurch gekennzeichnet, dass** die Vorrichtung eine Anzahl von in der Ringleitung (22) integrierten Ventilen (13, 14, 15) aufweist, wobei die Ventile (13, 14, 15) derart in der Ringleitung (22) angeordnet und ausgebildet sind, dass eine automatische Reinigung der Vorrichtung, insbesondere der Ringleitung, und/oder eine Spülung mit Spülgas, insbesondere Stickstoff, und/oder eine Befüllung der Ringleitung (22) und/oder Entnahme von Fluiden über die Ventile (13, 14, 15) erfolgen kann.

15. Vorrichtung nach einem der Ansprüche 11 bis 14, **dadurch gekennzeichnet, dass** der Piercer (2) hohl ausgebildet ist, wobei das Entnahmerohr (3) durch den Piercer (2) hindurch in die Entnahmeöffnung einbringbar ist.

Fig. 1

**Europäisches Patentamt**
**European Patent Office**
**Office européen des brevets**

# EUROPÄISCHER RECHERCHENBERICHT

Nummer der Anmeldung

EP 20 16 3897

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP H10 7115 A (DAIWA CAN CO LTD) 13. Januar 1998 (1998-01-13) | 1,3,4, 11-14 | INV. G01N1/22 |
| Y | * Zusammenfassung; Abbildung 1 * * Absätze [0001], [0026] - [0034] * ----- | 2,5-10, 15 | G01N33/14 |
| Y | WO 2009/050530 A1 (HACH ULTRA ANALYTICS SA [CH]; STEHLE GERARD [FR] ET AL.) 23. April 2009 (2009-04-23) * Abbildungen 1-4 * * Seite 3, Zeile 16 - Seite 5, Zeile 14 * * Seite 5, Zeile 22 - Seite 9, Zeile 6 * ----- | 2,5-10 | |
| Y | US 2005/223822 A1 (OZBAL CAN [US]) 13. Oktober 2005 (2005-10-13) * Abbildungen 2-3 * * Absätze [0031] - [0032] * ----- | 15 | |
| A | EP 1 887 344 A1 (KIRIN BREWERY [JP]) 13. Februar 2008 (2008-02-13) * Abbildungen 1-7 * * Absatz [0030] - Absatz [0037] * ----- | 1-15 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| Den Haag | 7. September 2020 | Bockstahl, Frédéric |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 16 3897

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-09-2020

| Im Recherchenbericht angeführtes Patentdokument | Datum der Veröffentlichung | Mitglied(er) der Patentfamilie | Datum der Veröffentlichung |
|---|---|---|---|
| JP H107115 A | 13-01-1998 | JP H107115 A<br>JP 3577391 B2 | 13-01-1998<br>13-10-2004 |
| WO 2009050530 A1 | 23-04-2009 | CN 101821615 A<br>EP 2198289 A1<br>JP 2011501148 A<br>US 2010236320 A1<br>WO 2009050530 A1 | 01-09-2010<br>23-06-2010<br>06-01-2011<br>23-09-2010<br>23-04-2009 |
| US 2005223822 A1 | 13-10-2005 | CA 2562424 A1<br>EP 1740924 A1<br>JP 4405552 B2<br>JP 2007532877 A<br>US 2005223822 A1<br>WO 2005100945 A1 | 27-10-2005<br>10-01-2007<br>27-01-2010<br>15-11-2007<br>13-10-2005<br>27-10-2005 |
| EP 1887344 A1 | 13-02-2008 | EP 1887344 A1<br>JP 4358785 B2<br>JP 2007198735 A<br>US 2009084156 A1<br>WO 2006126683 A1 | 13-02-2008<br>04-11-2009<br>09-08-2007<br>02-04-2009<br>30-11-2006 |

EPO FORM P0461

Für nähere Einzelheiten zu diesem Anhang : siehe Amtsblatt des Europäischen Patentamts, Nr.12/82